# EUROPEAN PATENT SPECIFICATION

(11) **EP 3 353 752 B1**
(45) Date of publication and mention of the grant of the patent: **04.08.2021**
(21) Application number: 16779259.7
(22) Date of filing: 25.09.2016
(51) Int. Cl.: G06T 19/20

(54) **ADJUSTABLE DEPTH ANATOMICAL SHELL EDITING**
BEARBEITUNG EINER ANATOMISCHEN HÜLLE MIT VERSTELLBARER TIEFE
ÉDITION D'ENVELOPPE ANATOMIQUE À PROFONDEUR AJUSTABLE

(30) Priority: 26.09.2015 US 201562233348 P
(43) Date of publication of application: 01.08.2018
(73) Proprietor: Boston Scientific Scimed Inc., Maple Grove, Minnesota 55311 (US)
(72) Inventor: PERLMAN, Mordechai, Cambridge, Massachusetts 02138 (US); KLEBANOV, Daniel, Arlington, Massachusetts 02474 (US); HRISTOV, Ruslan R., Lexington, Massachusetts 02420 (US)
(74) Representative: Pfenning, Meinig & Partner mbB
(86) International application number: PCT/US2016/053630
(87) International publication number: WO 2017/053924

(56) References cited:
- EP-A1- 2 204 120
- EP-A2- 2 485 194
- EP-A2- 2 680 234
- WO-A2-2004/008640
- WO-A2-2007/108776
- US-A- 4 835 688
- US-A1- 2008 319 308
- US-A1- 2014 200 874
- Andras Lasso ET AL: "SlicerWiki VolumeClip", , 25 December 2014 (2014-12-25), pages 1-4, XP055332376, Retrieved from the Internet: URL:https://www.slicer.org/wiki/Documentat ion/4.4/Extensions/VolumeClip [retrieved on 2017-01-05]
- Anonymous: "Solid commands | Rhino 3-D modeling (Rhinoceros 5)", , 17 September 2015 (2015-09-17), XP55332631, Retrieved from the Internet: URL:http://docs.mcneel.com/rhino/5/help/en -us/seealso/sak_solidtools.htm [retrieved on 2017-01-05]

## Description

### CROSS-REFERENCE TO RELATED APPLICATION

This application claims priority to Provisional Application No. 62/233,348, filed September 26, 2015.

### TECHNICAL FIELD

The present disclosure generally relates to cardiac mapping systems and methods. More specifically, the present disclosure relates to editing anatomical shells.

### BACKGROUND

Diagnosing and treating cardiac disorders often involve the introduction of a catheter into a cardiac chamber through the surrounding vasculature. The catheter has a plurality of sensors, located on the catheter's distal end. Information received from the plurality of sensors can be used to construct an anatomical shell, which is a graphical representation of the cardiac chamber. The anatomical shell can be used by a physician or other medical professional for treating cardiac disorders.

When constructing the anatomical shell of a cardiac chamber, various issues can occur. For example, in some instances, the catheter can be pushed against the wall of the cardiac chamber, which stretches the chamber and results in a slightly distorted version of the chamber. As another example, some cardiac protrusions that project inwardly from the endocardium surface of the cardiac chamber are not shown on the anatomical shell.

Conventional systems may allow for manual editing of the anatomical shell, which may include selecting a region of the surface of the anatomical shell to remove. Removal of the selected region of the surface also causes all of the points that lay below that region of the surface to be removed, to an infinite depth - that is, any points that lay inward the selected region of the surface are also removed. This may result in the undesirable removal of regions of the surface of the anatomical shell that lie inward the selected region and leaves a hole in the anatomical shell. Document US 4 835 688 A relates to a three-dimensional image processing apparatus including a first three-dimensional memory for storing first voxel data representing a three-dimensional CT image, a level detector for comparing the first voxel data with CT values of an epidermis, a bone, and a blood vessel, second three-dimensional memories for storing second voxel data (binary) of the epidermis, the bone, and the blood vessel obtained by the level detector, a distance detector for detecting distances between a predetermined projection plane in a three-dimensional space and the second voxel data, a minimum detector for detecting a distance between the projection plane and one of the second voxel data of the epidermis, bone, and blood vessel, which is closest to the projection plane, and a surface image generating circuit for shading minimum values of pixels in the projection plane to generate a surface image.

Document EP-A-2 204 120 relates to a multiple shell construction to emulate chamber contraction with a mapping system.

### SUMMARY

Embodiments of the subject matter disclosed herein include cardiac mapping systems that allow a user to perform adjustable depth anatomical shell editing. The invention is set out in the appended set of claims.

A processor-implemented method for adjustable depth anatomical shell editing comprises: outputting, to a display device, an anatomical shell of a cardiac structure, wherein the anatomical shell is based on a plurality of signals sensed by a mapping probe; receiving, from a user input device, a selection of a region of the surface of the anatomical shell; generating a modified anatomical shell by removing a portion of the anatomical shell, the removed portion of the anatomical shell comprising the selected region of the surface and extending inward to an editing depth, the editing depth comprising at least one finite depth; and outputting, to the display device, the modified anatomical shell.

The generating the modified anatomical shell comprises generating a new surface region on the modified anatomical shell, corresponding to the selected region, wherein the new surface region is disposed at the editing depth.

Receiving, from the user input device, a command to undo the generation of the modified anatomical shell; regenerating the anatomical shell; and outputting the regenerated anatomical shell to the display device.

Receiving, from the user input device, a user input specifying a target depth, wherein the editing depth is determined based on the target depth.

A target depth is at least 1 millimeter and at most 5 millimeters.

Receiving, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

Receiving, from the user input device, a command to display a selection tool on the user interface; and receiving, from the user input device, a selection of a size of the selection tool.

A mapping system comprises: a display device configured to display an anatomical shell of a cardiac structure based on a plurality of signals sensed by a mapping probe; a user input device configured to receive input from a user; and a processing device coupled to the display device and the user input device, wherein the processing device is configured to: receive, from the user input device, a selection of a region of the surface of the anatomical shell; generate a modified anatomical shell by removing a portion of the anatomical shell, the removed portion of the anatomical shell comprising the selected region of the surface and extending inward to an editing depth, the editing depth comprising at least one finite depth; and output the modified anatomical shell to the display device.

A processing device is further configured to generate a new surface region on the modified anatomical shell, corresponding to the selected region, wherein the new surface region is disposed at the editing depth.

A processing device is further configured to: receive, from the user input device, a command to undo the generation of the modified anatomical shell; regenerate the anatomical shell; and output the regenerated anatomical shell to the display device.

A processing device is further configured to receive, from the user input device, a user input specifying a target depth, wherein the editing depth is determined based on the target depth.

A target depth is at least 1 millimeter and at most 5 millimeters.

A processing device is configured to determine the editing depth based on the target depth and a contour of the selected region of the surface of the anatomical shell.

A processing device is further configured to: receive, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

A processing device is further configured to: receive, from the user input device, a command to display a selection tool on the user interface; and receive, from the user input device, a selection of a size of the selection tool.

A processor-implemented method for adjustable depth anatomical shell editing, the method comprising: receiving a plurality of signals sensed by a mapping probe; generating, using the processor, an anatomical shell of a cardiac structure, wherein the anatomical shell is generated based on the plurality of signals; outputting, to a display device, the anatomical shell of the cardiac structure; receiving, from a user input device, a selection of a region of the surface of the anatomical shell; generating a modified anatomical shell by removing a portion of the anatomical shell, the removed portion of the anatomical shell comprising the selected region of the surface and extending inward to an editing depth, the editing depth comprising at least one finite depth; and outputting, to the display device, the modified anatomical shell.

The generating the modified anatomical shell comprises generating a new surface region on the modified anatomical shell, corresponding to the selected region, wherein the new surface region is disposed at the editing depth.

Receiving, from the user input device, a command to undo the generation of the modified anatomical shell; regenerating the first anatomical shell; and outputting the regenerated anatomical shell to the display device.

Receiving, from the user input device, a user input specifying a target depth, wherein the editing depth is determined based on the target depth.

A target depth is at least 1 millimeter and at most 5 millimeters.

Determining the editing depth based on the target depth and a contour of the selected region of the surface of the anatomical shell.

Receiving, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

Receiving, from the user input device, a command to display a selection tool on the user interface; and receiving, from the user input device, a selection of a size of the selection tool.

A mapping system comprising: a display device configured to display an anatomical shell of a cardiac structure based on a plurality of signals sensed by a mapping probe; a user input device configured to receive input from a user; and a processing device coupled to the display device and the user input device, wherein the processing device is configured to: receive, from the user input device, a selection of a region of the surface of the anatomical shell; generate a modified anatomical shell by removing a portion of the anatomical shell, the removed portion of the anatomical shell comprising the selected region of the surface and extending inward to an editing depth, the editing depth comprising at least one finite depth; and output the modified anatomical shell to the display device.

A processing device is further configured to generate a new surface region on the modified anatomical shell, corresponding to the selected region, wherein the new surface region is disposed at the editing depth.

A processing device is further configured to: receive, from the user input device, a command to undo the generation of the modified anatomical shell; regenerate the anatomical shell; and output the regenerated anatomical shell to the display device.

A processing device is further configured to receive, from the user input device, a user input specifying a target depth, wherein the editing depth is determined based on the target depth.

A target depth is at least 1 millimeter and at most 5 millimeters.

A processing device is configured to determine the editing depth based on the target depth and a contour of the selected region of the surface of the anatomical shell.

A processing device is further configured to: receive, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

A processing device is further configured to: receive, from the user input device, a command to display a selection tool on the user interface; and receive, from the user input device, a selection of a size of the selection tool.

One or more computer-readable media having computer-executable instructions embodied thereon that, when executed by a processor, cause the processor to: output, to a display device, an anatomical shell of a cardiac structure, wherein the anatomical shell is based on a plurality of signals sensed by a mapping probe; receive, from a user input device, a selection of a region of the surface of the anatomical shell; receive, from the user input device, a user input specifying a target depth, wherein the editing depth is determined based on the target depth; receive, from the user input device, a command to remove a portion of the anatomical shell; generate a modified anatomical shell by removing the portion of the anatomical shell, the removed portion of the anatomical shell comprising the selected region of the surface and extending inward to an editing depth, the editing depth comprising at least one finite depth; and output, to the display device, the modified anatomical shell.

The instructions further cause the processor to: receive, from the user input device, a command to undo the generation of the modified anatomical shell; regenerate the first anatomical shell; and output the regenerated anatomical shell to the display device.

The instructions further cause the processor to receive, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

The instructions further cause the processor to: receive, from the user input device, a command to display a selection tool on the user interface; and receive, from the user input device, a selection of a size of the selection tool.

### BRIEF DESCRIPTION OF THE DRAWINGS

FIG. 1 is a schematic view of a system for mapping and viewing internal cardiac structures, in accordance with embodiments of the present disclosure.
FIG. 2 is a schematic view of a mapping probe for use in association with the system of FIG. 1, in accordance with embodiments of the disclosure.
FIG. 3 is a flow diagram depicting an illustrative processor-implemented method for adjustable depth anatomical shell editing, in accordance with the disclosure.
FIGS. 4A-4B are images of an anatomical shell shown from different perspectives, in accordance with embodiments of the disclosure.
FIGS. 5A-5B are images of another anatomical shell shown from different perspectives, in accordance with embodiments of the disclosure.

While the disclosed subject matter is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

Although the term "block" may be used herein to connote different elements illustratively employed, the term should not be interpreted as implying any requirement of, or particular order among or between, various steps disclosed herein unless and except when explicitly referring to the order of individual steps.

### DETAILED DESCRIPTION

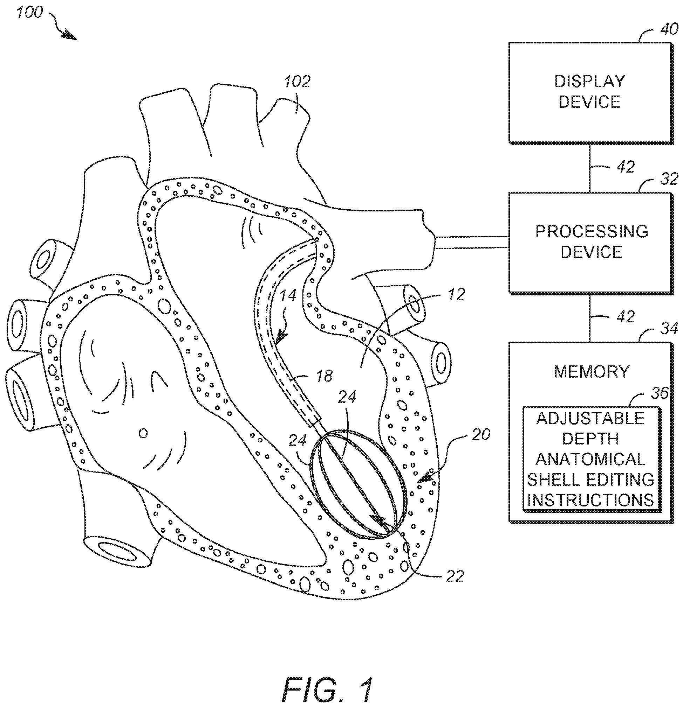

FIG. 1 is a schematic view of a mapping system 100 for mapping cardiac structures 102, in accordance with embodiments of the disclosure. Throughout this disclosure, the term "cardiac structure" can mean any portion of a subject's heart and/or adjacent features such as, for example, an endocardium, an epicardium, an entire heart, a heart chamber, a portion of a heart chamber, a valve, a coronary sinus and/or its tributaries, a portion of a coronary sinus and/or a portion of its tributaries, a pulmonary artery, other surrounding vasculature, and/or the like. While this disclosure discusses using the mapping system to map cardiac structures 102, the mapping system 100 may also, or alternatively, be used to map other organs and/or biological tissue including, but not limited to, kidneys, lungs, brains, gall bladders, livers, spleens, intestines, and/or the like.

The system 100 includes a mapping probe 14. The mapping probe 14 includes a flexible catheter body 18. When mapping cardiac structures 102, a physician or medical professional inserts the distal end of the catheter body 18 into a cardiac chamber (e.g., the left ventricle of the heart) of a patient. While the left ventricle of the heart is shown, alternatively, the distal end of the catheter body 18 can be deployed in other parts of the heart and/or surrounding vasculature, such as, e.g., the left atrium, the right atrium, or the right ventricle, the coronary sinus and its tributaries and the pulmonary artery. The distal end of the catheter body 18 has a multiple electrode structure 20. In the illustrated embodiments, the electrode structure 20 takes the form of a basket defining an open interior space 22. While the electrode structure 20 takes the form of a basket in the illustrated embodiments, this is only an example and other electrode structures can be utilized. For example, the electrode structure may include one or more electrodes (e.g., ablation electrodes, microelectrodes, ring electrodes, etc.) disposed on an ablation catheter, a diagnostic catheter, and/or the like.

As shown in FIG. 1, the electrode structure 20 includes a number of electrodes 24. The electrodes 24 are configured to sense electrical signals traversing the cardiac structure 102. The electrical signals can be sensed at any number of various locations in the patient's heart such as, for example, on the endocardium surface of the heart and/or in the heart chamber below the endocardium surface. As used herein, a mapped representation of a signal that is sensed at a point inside a heart chamber is referred to as being below the endocardium surface. That is, for example, because the map includes an anatomical shell that represents an endocardium surface and, from the perspective of the viewer, a point that is located inside the chamber, and not on the endocardium surface, would appear to be below the mapped surface.

Each signal may be associated with a set of respective position coordinates that correspond to the location at which the signal was sensed. The respective electrical signals can include, but are not limited to, voltage magnitudes, activation signals and changes in activation signals over a period of time. Each of the respective position coordinates may include three-dimensional Cartesian coordinates, polar coordinates, and/or the like. In embodiments, other coordinate systems can be used. In embodiments, an arbitrary origin is used and the respective position coordinates refer to positions in space relative to the arbitrary origin. Since, in embodiments, the signals may be sensed on the endocardium surface as well as in the chamber enclosed by the endocardium surface, the respective position coordinates may be on the endocardium surface of the patient's heart and/or below the endocardium surface.

The electrodes 24 are electrically coupled to a processing device 32. That is, each electrode 24 on the basket structure 20 may be communicatively coupled to the processing device 32, via a wired and/or wireless connection. In embodiments where there is a wired connection, the wires (not shown) from each electrode may extend through the catheter body 18 of the mapping probe 14 and electrically couple each electrode 24 to the processing device 32. In embodiments where there is a wireless connection, a transmitter (not shown) may be included in the mapping probe 14 which may transmit sensed signals from each electrode 24 on the basket structure 20 to a receiver (not shown) that is coupled to the processing device 32.

Once the sensed signals are received by the processing device 32 from the electrodes 24, the processing device 32 processes the sensed signals. The processing device 32 processes the sensed signals according to adjustable depth anatomical shell editing instructions 36, which are stored on memory 34. The processing device 32 may be, include, or be included in, an electrical processor, a software processor, a general purpose microprocessor, and/or a special purpose microprocessor, and may include a sole processor or one of multiple processors or cores. The processed signals are displayed on a display device 40. The display device 40 may include, but is not limited to, one of the following display devices: a cathode ray tube (CRT) display, a light emitting diode (LED) display, and/or a liquid crystal display (LCD) display.

The memory 34 may be in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a processing device 32 such as, for example, quantum state memory, and/or the like. Mapping instructions 36 may be programmed on the memory 34 using any number of different programming environments, including various languages, development kits, frameworks, and/or the like. Some or all of the functionality contemplated herein may also, or alternatively, be implemented in hardware and/or firmware.

The processing device 32, the memory 34 and the display device 40 can be coupled together, directly and/or indirectly, by a bus 42. Any number of additional components, different components, and/or combinations of components may also be coupled to the processing device 32, memory 34 and display device 40, via the bus 42. The bus represents what may be one or more busses (such as, for example, an address bus, data bus, or combination thereof).

The illustrative mapping system 100 shown in FIG. 1 is not intended to suggest any limitation as to the scope of use or functionality of the present disclosure. Neither should the illustrative mapping system 100 be interpreted as having any dependency or requirement related to any single component or combination of components illustrated therein. Additionally, various components depicted in FIG. 1 may be, integrated with various ones of the other components depicted therein (and/or components not illustrated), all of which are considered to be within the ambit of the present disclosure. For example, the memory 34 may be integrated with the processing device 32.

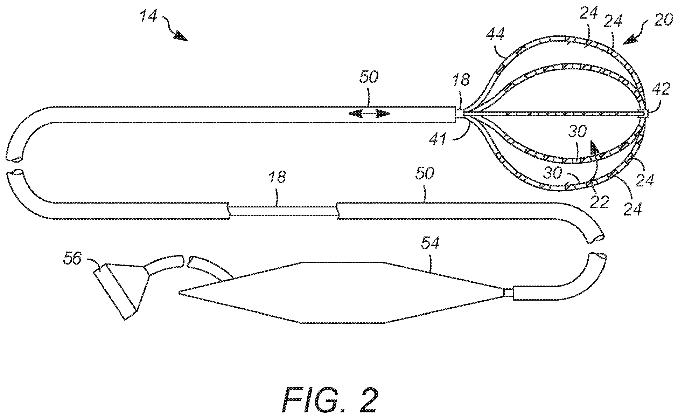

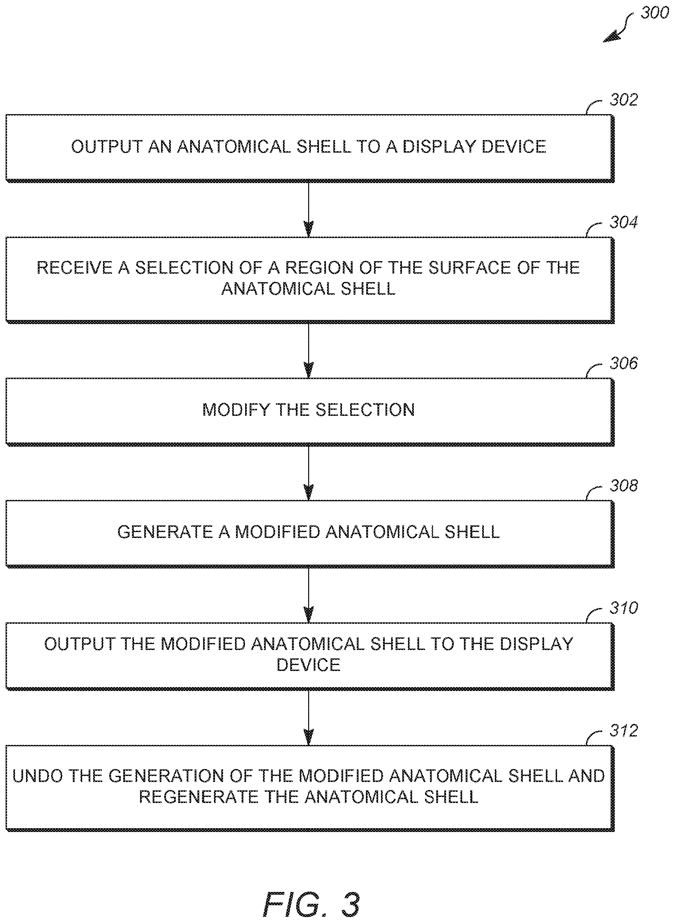

FIG. 2 is a schematic view of a mapping probe 14 for use in association with the system 100 of FIG. 1, in accordance with embodiments of the disclosure. The mapping probe 14 has a flexible catheter body 18, the distal end of which carries the three-dimensional basket structure 20 that includes the mapping electrodes 24. As stated above, the mapping electrodes 24 sense signals in a cardiac structure; and the sensed signals are sent to a processing device 32, via a wired and/or wireless connection. The processing device 32 then processes the sensed signals and an anatomical shell is created, as described, for example, in the description corresponding to FIG. 3, below.

The basket structure 20 comprises a base member 41 and an end cap 42 between which flexible splines 44 generally extend in a circumferentially spaced relationship. As discussed above, the basket structure 20 takes the form of a basket defining an open interior space 22. In embodiments, the splines 44 are made of a resilient inert material, such as Nitinol metal or silicone rubber, and are connected between the base member 41 and the end cap 42 in a resilient, pre-tensed condition, to bend and conform to the tissue surface they contact. In the illustrated embodiments, eight splines 44 form the three-dimensional structure 20. Additional or fewer splines 44 could be used in other embodiments, and the three-dimensional structure 20 may be configured according to any number of different shapes such as, for example, generally spherical shapes, generally elliptical shapes, generally teardrop shapes, and/or the like. As illustrated, each spline 44 carries eight mapping electrodes 24. Additional or fewer mapping electrodes 24 could be disposed on each spline 44 in other embodiments of the three-dimensional structure 20. In the illustrated embodiments, the three-dimensional structure 20 is relatively small (e.g., 40 mm or less in diameter). In embodiments, the three-dimensional structure 20 is larger (e.g., 40 mm in diameter or greater).

In embodiments, a slidable sheath 50 is movable along the major axis of the catheter body 30. Moving the sheath 50 forward (i.e., toward the distal end) causes the sheath 50 to move over the three-dimensional structure 20, thereby collapsing the structure 20 into a compact, low profile condition suitable for introduction into an interior space, such as, for example, into the heart. In contrast, moving the sheath 50 rearward (i.e., toward the proximal end) exposes the three-dimensional structure 20, allowing the structure 20 to elastically expand and assume the pre-tensed position illustrated in FIG. 2. Further details of embodiments of the three-dimensional structure 20 are disclosed, for example, in U.S. Pat. No. 5,647,870, entitled "Multiple Electrode Support Structures,".

In embodiments where the mapping probe 14 uses a wired connection, a signal wire (not shown) may be electrically coupled to each mapping electrode 24. The wires may extend through the body 30 of the mapping catheter 20 into a handle 54, in which they may be coupled to an external connector 56, which may be, for example, a multiple pin connector. The connector 56 electrically couples the mapping electrodes 24 to the processing system 32. Further details on mapping systems and methods for processing signal generated by mapping catheters are discussed, for example, in U.S. Pat. No. 6,070,094, entitled "Systems and Methods for Guiding Movable Electrode Elements within Multiple-Electrode Structure;" U.S. Pat. No. 6,233,491, entitled "Cardiac Mapping and Ablation Systems;" and U.S. Pat. No. 6,735,465, entitled "Systems and Processes for Refining a Registered Map of a Body Cavity,".

It is noted that other electrode structures could be deployed on the distal end of a mapping catheter. It is further noted that the multiple mapping electrodes 24 may be disposed on more than one structure, rather than, for example, the single mapping probe 14 illustrated in FIG. 2. For example, if mapping within the left atrium with multiple mapping structures, an arrangement comprising a coronary sinus catheter carrying multiple mapping electrodes and a basket catheter carrying multiple mapping electrodes positioned in the left atrium may be used. As another example, if mapping within the right atrium with multiple mapping structures, an arrangement comprising a decapolar catheter carrying multiple mapping electrodes for positioning in the coronary sinus, and a loop catheter carrying multiple mapping electrodes for positioning around the tricuspid annulus may be used.

Although the mapping electrodes 24 have been described as being carried by dedicated mapping probes, such as the mapping probe 14, the mapping electrodes may be carried on non-mapping dedicated probes or multifunction probes. For example, an ablation catheter can be configured to include one or more mapping electrodes 24 disposed on the distal end of the catheter body and coupled to the signal processing system 32.

FIG. 3 is a flow diagram depicting an illustrative processor-implemented method 300 for adjustable depth anatomical shell editing, in accordance with the disclosure. According to the invention the method 300 include outputting an anatomical shell to a display device (block 302). The anatomical shell may be generated based on a number of signals sensed by a mapping probe. In embodiments, the mapping probe can have the same or similar characteristics to the mapping probe 14 described above in FIGS. 1 and 2. Each of the respective signals sensed by the mapping probe has a corresponding set of three-dimensional position coordinates. For example, the position coordinates of the points may be represented in Cartesian coordinates. However, other coordinate systems may be used. In embodiments, an arbitrary origin is used and the respective position coordinates are defined with respect to the arbitrary origin. In some embodiments, the points have non-uniform spacing, while in other embodiments, the points have uniform spacing. According to the invention, the point corresponding to each sensed signal may be located on the endocardium surface of the heart and/or below the endocardium surface of the heart.

The anatomical shell may be generated, at least in part, using any number of other signals, techniques, and/or the like. For example, embodiments may utilize impedance mapping techniques to generate the anatomical shell. In embodiments, a surface may be fitted on one or more of the points associated with the electrical signals to generate a shell representing the endocardium surface. In embodiments, one or more features of the electrical signals at the corresponding points can be included in the shell of the endocardium surface. For example, embodiments may include displaying annotations on the shell that represent features extracted from the electrical signals such as, for example, voltages, activation amplitudes, signal sharpness and/or the like. As another example, a surface may be fitted on one or more of the points associated with the electrical signals to generate a shell representing an epicardium surface or other excitable cardiac tissue.

Embodiments of method 300 also include receiving, from a user input device, a selection of a region of the surface of the anatomical shell (block 304). The user input device used to make the selection may include a mouse, a touchscreen and/or the like, that is used to manipulate a selection tool that is provided on a user interface provided by the display device. The selection tool may include, for example, a brush, a cursor for enclosing the selected region by drawing a freeform shape around the region, an expandable polygon selection tool, and/or the like, and may be, in embodiments, selected from a number of optional selection tools. In embodiments, the selection tool may have an adjustable size, behavior and/or other characteristics thereof. In this manner, for example, a user may select a desired selection tool and a size thereof. Selecting a region of the surface of the anatomical shell may include, for example, circling the region of the surface of the anatomical shell using a mouse or touchscreen device to manipulate a cursor, brushing over the region of the surface using an input device to manipulate a brush, and/or the like.

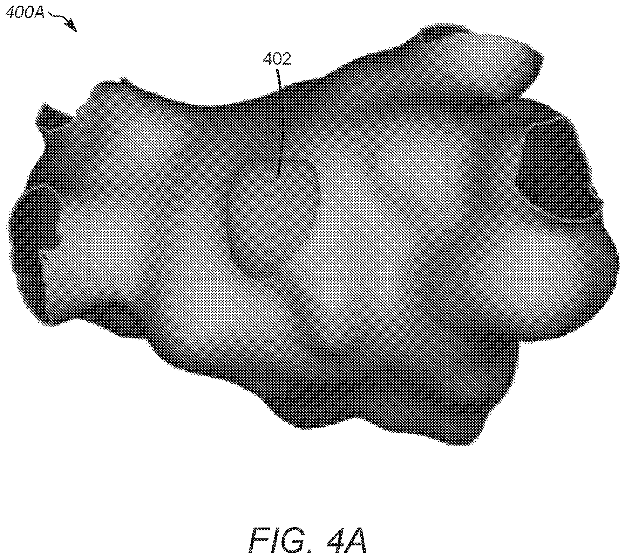

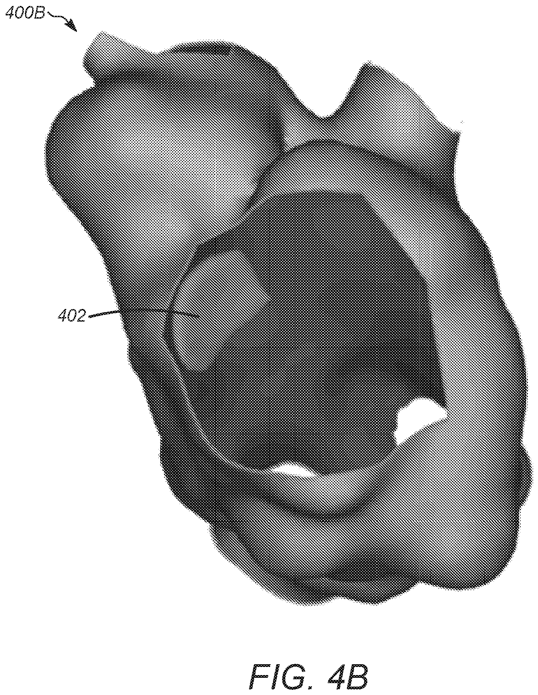

Examples of receiving selections of a portion of the first anatomical shell are shown in FIGS. 4A and 4B. In particular, FIG. 4A is a right anterior oblique view of an anatomical shell 400A that has a selected portion 402; and, FIG. 4B is a left lateral view of the anatomical shell with the same selected portion 402. In embodiments, method 300 may include steps for generating FIG. 4B from FIG. 4A, or vice-versa. That is, method 300 may include receiving, from a user input device, a command to rotate the displayed anatomical shell after the portion 402 is selected. After the command is received, method 300 can include generating a rotated view of the first anatomical shell based on the command.

In some embodiments, method 300 may also include receiving, from an input device, a modification to the selection of the region of the surface of the anatomical shell (block 306). A modification to the selection of the region is also referred to herein as a selection modification. In response to receiving the selection modification, the modified selection may be made of a region of the surface of the anatomical shell. Being able to modify the selection may be useful to a user that selected too big or too small of a region, for facilitating incremental selection, to facilitate more accurate selection, and/or the like. In embodiments, a modification to the selection may include a modification to the target depth of the selection, a modification to the size of the region of the surface selected, and/or the like. For example, in embodiments, the modification may include a selection of an additional region of the surface of the anatomical shell. As another example, in embodiments, the modification may include a selection of sub-region of the selected region of the surface of the anatomical shell. Similar to selecting a portion above, a mouse, touchscreen or the like can be used to modify the selection. In embodiments, modifying the selected portion may include deselecting the selected region and then reselecting a region. In embodiments, modifying the selection may include modifying the selection by altering the boundaries of the selection. In embodiments this may be done, for example, by clicking on a portion of the border of the selected region and dragging the selected portion of the border to a new location. In embodiments that include a selection modification, the removed portion of the anatomical shell (discussed below, block 308) can be the modified selected region.

Embodiments of method 300 also include generating a modified anatomical shell (block 308). According to the inventon, the modified anatomical shell is generated by removing a portion of the anatomical shell, where the removed portion of the anatomical shell includes the selected region (or modified selected region) of the surface and extending inward to an editing depth. The editing depth may include at least one finite depth and may vary, corresponding to the contour of the surface of the anatomical shell. That is, for example, the editing depth may be the distance, along an axis normal to the surface of the anatomical shell, between a point on the surface of the anatomical shell and a point inward from the surface (i.e., below the surface) of the anatomical shell. In that manner, the editing depth may be change with the contour of the surface.

According to embodiments, the editing depth may be adjustable and/or selectable. That is, for example, a user may input and/or select a target depth, via the user interface, such that the editing depth, corresponding to each point in the selected region, may be at least approximately equal to the target depth (e.g., equal to the target depth, within at least .1 millimeters of the target depth, within at least .5 millimeters of the target depth, within 1 millimeter of the target depth, and/or the like. In embodiments, the target depth may be less than one millimeter, at least one millimeter and no more than 10 millimeters, at least one millimeter and no more than 5 millimeters, and/or the like.

In this manner, the modified anatomical shell may have the same shape as the anatomical shell, except that a portion 402 corresponding to a selected region is removed to an editing depth. In embodiments, a command may be received from a user input device to remove the portion 402. In other embodiments, when the region is selected, the portion 402 may be automatically removed. In embodiments, generating a modified anatomical shell that has the portion 402 removed to an approximate depth from the first anatomical shell 400A, 400B may include generating a new surface region on the modified anatomical shell, corresponding to the selected region, wherein the new surface region is disposed at the editing depth. That is, for example, the contour of the selected region may remain the same but appear to be moved inward by the editing depth. As described above, the term "inward" refers to the direction opposite the normal of a point on the surface of the anatomical shell 400A, 400B.

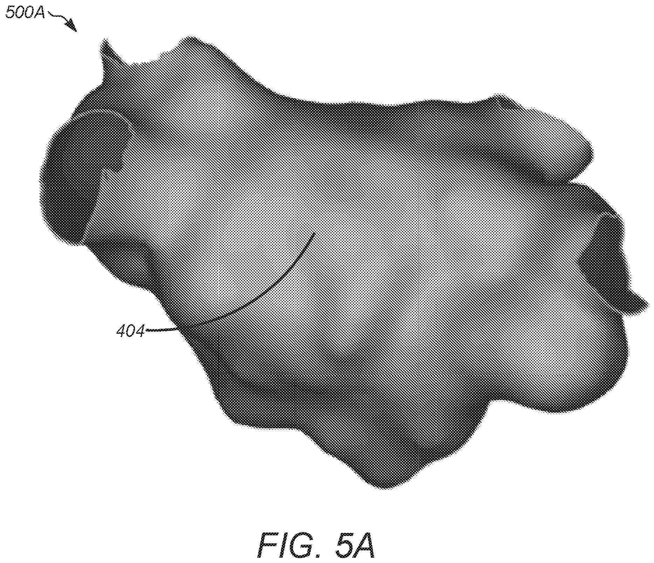

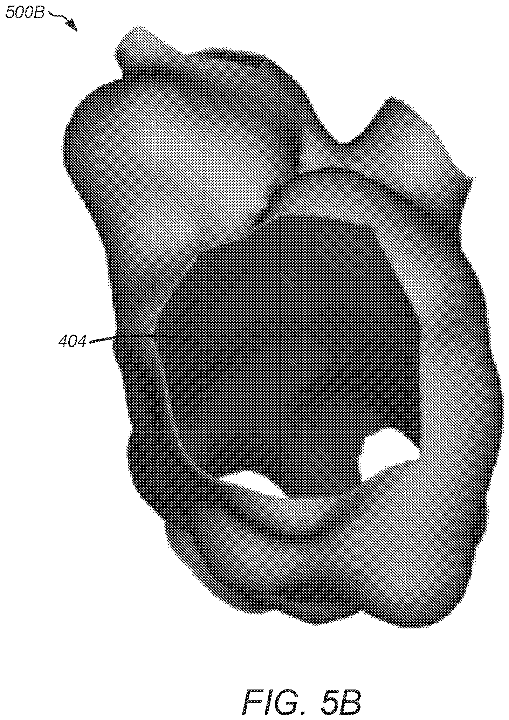

According to the invention the method 300 also include outputting the modified anatomical shell to the display device (block 310). FIGS. 5A, 5B are images of examples of generated modified anatomical shells 500A, 500B that were output to a display device. As can be seen in the images, the modified anatomical shells 500A, 500B include a new region 404 corresponding to the selected region 402, disposed at the editing depth. If the new region 404 is not generated on the modified anatomical shell 500A, 500B, the anatomical shell may include a hole to the editing depth where the selected region 402 is removed.

Embodiments of the method 300 may include receiving a command from a user input device to undo the generation of the modified anatomical shell. In response to receiving the command to undo the generation of the modified anatomical shell, the system may remove the modified anatomical shell and regenerate the anatomical shell (block 312). Similar to modifying the selected region in block 306, being able to undo generating the modified anatomical shell may be helpful if a user selects too big or too small of a region of the anatomical shell, selects a region that the user did not intend to select, and/or the like. The regenerated anatomical shell may be output to the display device for the user to view.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

## Claims

1. A processor-implemented method for adjustable depth anatomical shell editing, the method comprising:
outputting, to a display device (40), an anatomical shell of a cardiac structure (102), the cardiac structure comprising a heart chamber, wherein the anatomical shell (400A, 400B) is based on a plurality of electrical signals sensed by one or more electrodes (24) of a mapping probe (14);
receiving, from a user input device, a selection of a region of the surface of the anatomical shell (400A, 400B);
generating a modified anatomical shell (500A, 500B) by removing a portion (402) of the anatomical shell (400A, 400B) and generating a new surface region (404) on the modified anatomical shell (500A, 500B), the removed portion of the anatomical shell (400A, 400B) comprising the selected region of the surface and extending inward from the surface to an editing depth below the surface, the editing depth comprising at least one finite depth, and wherein the new surface region corresponds to the selected region disposed at the editing depth and comprises at least one electrical signal of the plurality of electrical signals sensed below an endocardium surface inside the heart chamber of the cardiac structure at the editing depth; and
outputting, to the display device, the modified anatomical shell (500A, 500B).

2. The method according to claim 1, further comprising:
receiving, from the user input device, a command to undo the generation of the modified anatomical shell (500A, 500B);
regenerating the anatomical shell (400A, 400B); and
outputting the regenerated anatomical shell to the display device.

3. The method according to any of claims 1 or 2, further comprising receiving, from the user input device, a user input specifying a target depth, wherein the editing depth is determined based on the target depth.

4. The method according to claim 3, wherein the target depth is at least 1 millimeter and at most 5 millimeters.

5. The method according to any of claims 1 - 4, further comprising:
receiving, from the user input device, a selection modification, wherein the selection modification comprises a modified selected region, the modified selected region comprising a sub-region of the selected region or the selected region and an additional region of the surface of the anatomical shell, wherein the removed portion of the anatomical shell comprises the modified selected region.

6. The method according to any of claims 1 - 5, further comprising:
receiving, from the user input device, a command to display a selection tool on the user interface; and
receiving, from the user input device, a selection of a size of the selection tool.

7. A mapping system (100), comprising:
a display device (40) configured to display an anatomical shell (400A, 400B) of a cardiac structure (102) based on a plurality of electrical signals sensed by one or more electrodes (24) of a mapping probe (14); the cardiac structure comprising a heart chamber;
a user input device configured to receive input from a user; and
a processing device (32) coupled to the display device and the user input device
wherein the processing device is configured to:
receive, from the user input device, a selection of a region of the surface of the anatomical shell (400A, 400B);
generate a modified anatomical shell (500A, 500B) by removing a portion (402) of the anatomical shell (400A, 400B) and generating a new surface region (404) on the modified anatomical shell (500A, 500B), the removed portion of the anatomical shell (400A, 400B) comprising the selected region of the surface and extending inward from the surface to an editing depth below the surface, the editing depth comprising at least one finite depth, and wherein the new surface region corresponds to the selected region disposed at the editing depth and comprises at least one electrical signal of the plurality of electrical signals sensed below an endocardium surface inside the heart chamber of the cardiac structure at the editing depth; and
output the modified anatomical (500A, 500B) shell to the display device.

8. The mapping system according to either claim 7, wherein the processing device is further configured to:
receive, from the user input device, a command to undo the generation of the modified anatomical shell (500A, 500B);
regenerate the anatomical shell (400A, 400B); and
output the regenerated anatomical shell to the display device.

9. The mapping system according to any of claims 7 or 8, wherein the processing device is further configured to receive, from the user input device, a user input specifying a target depth, wherein the editing depth is determined based on the target depth.

10. The mapping system according to claim 9, wherein the target depth is at least 1 millimeter and at most 5 millimeters.

11. The mapping system according to any of claims 7 - 10, wherein the processing device is configured to determine the editing depth based on the target depth and a contour of the selected region of the surface of the anatomical shell (400A, 400B).

12. The mapping system according to any of claims 7 - 11, wherein the processing device is further configured to:
receive, from the user input device, a selection modification, wherein the selection modification comprises a modified selected region, the modified selected region comprising a sub-region of the selected region or the selected region and an additional region of the surface of the anatomical shell (400A, 400B), wherein the removed portion of the anatomical shell (400A, 400B) comprises the modified selected region.

13. The mapping system according to any of claims 7 - 12, wherein the processing device is further configured to:
receive, from the user input device, a command to display a selection tool on the user interface; and
receive, from the user input device, a selection of a size of the selection tool.

## Patentansprüche

1. Prozessor-implementiertes Verfahren zur Bearbeitung einer anatomischen Hülle mit verstellbarer Tiefe, wobei das Verfahren umfasst:
Ausgeben, an eine Anzeigevorrichtung (40), einer anatomischen Hülle einer kardialen Struktur (102), wobei die kardiale Struktur eine Herzkammer umfasst, wobei die anatomische Hülle (400A, 400B) auf einer Vielzahl elektrischer Signale basiert, die von einer oder mehreren Elektroden (24) einer Abbildungssonde (14) erfasst wurden;
Empfangen, von einer Benutzereingabevorrichtung, einer Auswahl einer Region der Oberfläche der anatomischen Hülle (400A, 400B);
Erzeugen einer modifizierten anatomischen Hülle (500A, 500B) durch Entfernen eines Teils (402) der anatomischen Hülle (400A, 400B) und Erzeugen einer neuen Oberflächenregion (404) auf der modifizierten anatomischen Hülle (500A, 500B), wobei der entfernte Teil der anatomischen Hülle (400A, 400B) die ausgewählte Region der Oberfläche umfasst und sich von der Oberfläche zu einer Bearbeitungstiefe unter der Oberfläche nach innen erstreckt, wobei die Bearbeitungstiefe mindestens eine finite Tiefe umfasst, und wobei die neue Oberflächenregion der ausgewählten Region entspricht, die in der Bearbeitungstiefe angeordnet ist und mindestens ein elektrisches Signal von der Vielzahl von elektrischen Signalen umfasst, die unter einer Endokardfläche in der Herzkammer der kardialen Struktur in der Bearbeitungstiefe erfasst wurden; und
Ausgeben, an die Anzeigevorrichtung, der modifizierten anatomischen Hülle (500A, 500B).

2. Verfahren nach Anspruch 1, ferner umfassend:
Empfangen, von der Benutzereingabevorrichtung, eines Befehls, die Erzeugung der modifizierten anatomischen Hülle (500A, 500B) rückgängig zu machen;
Regenerieren der anatomischen Hülle (400A, 400B); und
Ausgeben der regenerierten anatomischen Hülle an die Anzeigevorrichtung.

3. Verfahren nach einem der Ansprüche 1 oder 2, ferner umfassend Empfangen, von der Benutzereingabevorrichtung, einer Benutzereingabe, die eine Zieltiefe spezifiziert, wobei die Bearbeitungstiefe basierend auf der Zieltiefe bestimmt wird.

4. Verfahren nach Anspruch 3, wobei die Zieltiefe mindestens 1 Millimeter und höchstens 5 Millimeter ist.

5. Verfahren nach einem der Ansprüche 1-4, ferner umfassend:
Empfangen, von der Benutzereingabevorrichtung, einer Auswahlmodifikation, wobei die Auswahlmodifikation eine modifizierte ausgewählte Region umfasst, wobei die modifizierte ausgewählte Region eine Teilregion der ausgewählten Region oder die ausgewählte Region und eine zusätzliche Region der Oberfläche der anatomischen Hülle umfasst, wobei der entfernte Teil der anatomischen Hülle die modifizierte ausgewählte Region umfasst.

6. Verfahren nach einem der Ansprüche 1-5, ferner umfassend:
Empfangen, von der Benutzereingabevorrichtung, eines Befehls zur Anzeige eines Auswahlwerkzeugs auf der Benutzeroberfläche; und
Empfangen, von der Benutzereingabevorrichtung, einer Auswahl einer Größe des Auswahlwerkzeugs.

7. Abbildungssystem (100), umfassend:
eine Anzeigevorrichtung (40), dazu ausgelegt, eine anatomische Hülle (400A, 400B) einer kardialen Struktur (102) basierend auf einer Vielzahl elektrischer Signale, die von einer oder mehreren Elektroden (24) einer Abbildungssonde (14) erfasst wurden, anzuzeigen; wobei die kardiale Struktur eine Herzkammer umfasst;
eine Benutzereingabevorrichtung, dazu ausgelegt, Eingabe von einem Benutzer zu empfangen; und
eine Verarbeitungsvorrichtung (32), gekoppelt an die Anzeigevorrichtung und die Benutzereingabevorrichtung, wobei die Verarbeitungsvorrichtung dazu ausgelegt ist:
von der Benutzereingabevorrichtung eine Auswahl einer Region der Oberfläche der anatomischen Hülle (400A, 400B) zu empfangen;
eine modifizierte anatomische Hülle (500A, 500B) durch Entfernen eines Teils (402) der anatomischen Hülle (400A, 400B) und Erzeugen einer neuen Oberflächenregion (404) auf der modifizierten anatomischen Hülle (500A, 500B) zu erzeugen, wobei der entfernte Teil der anatomischen Hülle (400A, 400B) die ausgewählte Region der Oberfläche umfasst und sich von der Oberfläche zu einer Bearbeitungstiefe unter der Oberfläche nach innen erstreckt, wobei die Bearbeitungstiefe mindestens eine finite Tiefe umfasst, und wobei die neue Oberflächenregion der ausgewählten Region entspricht, die in der Bearbeitungstiefe angeordnet ist und mindestens ein elektrisches Signal von der Vielzahl von elektrischen Signalen umfasst, die unter einer Endokardfläche in der Herzkammer der kardialen Struktur in der Bearbeitungstiefe erfasst wurden; und
die modifizierte anatomische Hülle (500A, 500B) an die Anzeigevorrichtung auszugeben.

8. Abbildungssystem nach Anspruch 7, wobei die Verarbeitungsvorrichtung ferner dazu ausgelegt ist:
von der Benutzereingabevorrichtung einen Befehl, die Erzeugung der modifizierten anatomischen Hülle (500A, 500B) rückgängig zu machen, zu empfangen;
die anatomische Hülle (400A, 400B) zu regenerieren; und
die regenerierte anatomische Hülle an die Anzeigevorrichtung auszugeben.

9. Abbildungssystem nach einem der Ansprüche 7 oder 8, wobei die Verarbeitungsvorrichtung ferner dazu ausgelegt ist, von der Benutzereingabevorrichtung, eine Benutzereingabe, die eine Zieltiefe spezifiziert, wobei die Bearbeitungstiefe basierend auf der Zieltiefe bestimmt wird, zu empfangen.

10. Abbildungssystem nach Anspruch 9, wobei die Zieltiefe mindestens 1 Millimeter und höchstens 5 Millimeter ist.

11. Abbildungssystem nach einem der Ansprüche 7-10, wobei die Verarbeitungsvorrichtung dazu ausgelegt ist, die Bearbeitungstiefe basierend auf der Zieltiefe und einer Kontur der ausgewählten Region der Oberfläche der anatomischen Hülle (400A, 400B) zu bestimmen.

12. Abbildungssystem nach einem der Ansprüche 7-11, wobei die Verarbeitungsvorrichtung ferner dazu ausgelegt ist:
von der Benutzereingabevorrichtung eine Auswahlmodifikation zu empfangen, wobei die Auswahlmodifikation eine modifizierte ausgewählte Region umfasst, wobei die modifizierte ausgewählte Region eine Teilregion der ausgewählten Region oder die ausgewählte Region und eine zusätzliche Region der Oberfläche der anatomischen Hülle (400A, 400B) umfasst, wobei der entfernte Teil der anatomischen Hülle (400A, 400B) die modifizierte ausgewählte Region umfasst.

13. Abbildungssystem nach einem der Ansprüche 7-12, wobei die Verarbeitungsvorrichtung ferner dazu ausgelegt ist:
von der Benutzereingabevorrichtung einen Befehl zur Anzeige eines Auswahlwerkzeugs auf der Benutzeroberfläche zu empfangen; und
von der Benutzereingabevorrichtung eine Auswahl einer Größe des Auswahlwerkzeugs zu empfangen.

## Revendications

1. Procédé mis en œuvre par un processeur pour éditer une enveloppe anatomique à profondeur réglable, le procédé comprenant :
l'émission en sortie, sur un dispositif d'affichage (40), d'une enveloppe anatomique d'une structure cardiaque (102), la structure cardiaque comprenant une chambre cardiaque, dans lequel l'enveloppe anatomique (400A, 400B) est basée sur une pluralité de signaux électriques qui sont détectés par une ou plusieurs électrode(s) (24) d'une sonde de cartographie (14) ;
la réception, en provenance d'un dispositif d'entrée d'utilisateur, d'une sélection d'une région de la surface de l'enveloppe anatomique (400A, 400B) ;
la génération d'une enveloppe anatomique modifiée (500A, 500B) en supprimant une partie (402) de l'enveloppe anatomique (400A, 400B) et en générant une nouvelle région de surface (404) sur l'enveloppe anatomique modifiée (500A, 500B), la partie supprimée de l'enveloppe anatomique (400A, 400B) comprenant la région sélectionnée de la surface et étant étendue vers l'intérieur depuis la surface jusqu'à une profondeur d'édition en dessous de la surface, la profondeur d'édition comprenant au moins une profondeur finie, et dans lequel la nouvelle région de surface correspond à la région sélectionnée qui est disposée au niveau de la profondeur d'édition et comprend au moins un signal électrique de la pluralité de signaux électriques qui sont détectés en dessous d'une surface endocarde à l'intérieur de la chambre cardiaque de la structure cardiaque à la profondeur d'édition ; et
l'émission en sortie, sur le dispositif d'affichage, de l'enveloppe anatomique modifiée (500A, 500B).

2. Procédé selon la revendication 1, comprenant en outre :
la réception, en provenance du dispositif d'entrée d'utilisateur, d'une commande pour réaliser la génération de l'enveloppe anatomique modifiée (500A, 500B) ;
la régénération de l'enveloppe anatomique (400A, 400B) ; et
l'émission en sortie de l'enveloppe anatomique régénérée sur le dispositif d'affichage.

3. Procédé selon l'une quelconque des revendications 1 ou 2, comprenant en outre la réception, en provenance du dispositif d'entrée d'utilisateur, d'une entrée d'utilisateur qui spécifie une profondeur cible, dans lequel la profondeur d'édition est déterminée sur la base de la profondeur cible.

4. Procédé selon la revendication 3, dans lequel la profondeur cible est d'au moins 1 millimètre et d'au plus 5 millimètres.

5. Procédé selon l'une quelconque des revendications 1 à 4, comprenant en outre :
la réception, en provenance du dispositif d'entrée d'utilisateur, d'une modification de sélection, dans lequel la modification de sélection comprend une région sélectionnée modifiée, la région sélectionnée modifiée comprenant une sous-région de la région sélectionnée ou la région sélectionnée et une région additionnelle de la surface de l'enveloppe anatomique, dans lequel la partie supprimée de l'enveloppe anatomique comprend la région sélectionnée modifiée.

6. Procédé selon l'une quelconque des revendications 1 à 5, comprenant en outre :
la réception, en provenance du dispositif d'entrée d'utilisateur, d'une commande pour afficher un outil de sélection sur l'interface utilisateur; et
la réception, en provenance du dispositif d'entrée d'utilisateur, d'une sélection d'une dimension de l'outil de sélection.

7. Système de cartographie (100), comprenant :
un dispositif d'affichage (40) qui est configuré pour afficher une enveloppe anatomique (400A, 400B) d'une structure cardiaque (102) sur la base d'une pluralité de signaux électriques qui sont détectés par une ou plusieurs électrode(s) (24) d'une sonde de cartographie (14) ; la structure cardiaque comprenant une chambre cardiaque ;
un dispositif d'entrée d'utilisateur qui est configuré pour recevoir une entrée en provenance d'un utilisateur ; et
un dispositif de traitement (32) qui est couplé au dispositif d'affichage et au dispositif d'entrée d'utilisateur, dans lequel le dispositif de traitement est configuré pour :
recevoir, en provenance du dispositif d'entrée d'utilisateur, une sélection d'une région de la surface de l'enveloppe anatomique (400A, 400B) ;
générer une enveloppe anatomique modifiée (500A, 500B) en supprimant une partie (402) de l'enveloppe anatomique (400A, 400B) et en générant une nouvelle région de surface (404) sur l'enveloppe anatomique modifiée (500A, 500B), la partie supprimée de l'enveloppe anatomique (400A, 400B) comprenant la région sélectionnée de la surface et étant étendue vers l'intérieur depuis la surface jusqu'à une profondeur d'édition en dessous de la surface, la profondeur d'édition comprenant au moins une profondeur finie, et dans lequel la nouvelle région de surface correspond à la région sélectionnée qui est disposée au niveau de la profondeur d'édition et comprend au moins un signal électrique de la pluralité de signaux électriques qui sont détectés en dessous d'une surface endocarde à l'intérieur de la chambre cardiaque de la structure cardiaque à la profondeur d'édition ; et
émettre en sortie l'enveloppe anatomique modifiée (500A, 500B) sur le dispositif d'affichage.

8. Système de cartographie selon la revendication 7, dans lequel le dispositif de traitement est en outre configuré pour :
recevoir, en provenance du dispositif d'entrée d'utilisateur, une commande pour réaliser la génération de l'enveloppe anatomique modifiée (500A, 500B) ;
régénérer l'enveloppe anatomique (400A, 400B) ; et
émettre en sortie l'enveloppe anatomique régénérée sur le dispositif d'affichage.

9. Système de cartographie selon l'une quelconque des revendications 7 ou 8, dans lequel le dispositif de traitement est en outre configuré pour recevoir, en provenance du dispositif d'entrée d'utilisateur, une entrée d'utilisateur qui spécifie une profondeur cible, dans lequel la profondeur d'édition est déterminée sur la base de la profondeur cible.

10. Système de cartographie selon la revendication 9, dans lequel la profondeur cible est d'au moins 1 millimètre et d'au plus 5 millimètres.

11. Système de cartographie selon l'une quelconque des revendications 7 à 10, dans lequel le dispositif de traitement est configuré pour déterminer la profondeur d'édition sur la base de la profondeur cible et d'un contour de la région sélectionnée de la surface de l'enveloppe anatomique (400A, 400B).

12. Système de cartographie selon l'une quelconque des revendications 7 à 11, dans lequel le dispositif de traitement est en outre configuré pour :
recevoir, en provenance du dispositif d'entrée d'utilisateur, une modification de sélection, dans lequel la modification de sélection comprend une région sélectionnée modifiée, la région sélectionnée modifiée comprenant une sous-région de la région sélectionnée ou la région sélectionnée et une région additionnelle de la surface de l'enveloppe anatomique (400A, 400B), dans lequel la partie supprimée de l'enveloppe anatomique (400A, 400B) comprend la région sélectionnée modifiée.

13. Système de cartographie selon l'une quelconque des revendications 7 à 12, dans lequel le dispositif de traitement est en outre configuré pour :
recevoir, en provenance du dispositif d'entrée d'utilisateur, une commande pour afficher un outil de sélection sur l'interface utilisateur ; et
recevoir, en provenance du dispositif d'entrée d'utilisateur, une sélection d'une dimension de l'outil de sélection.
